(19) Europäisches Patentamt
European Patent Office
Office européen des brevets

(11) Publication number : 0 508 835 A2

(12) EUROPEAN PATENT APPLICATION

(21) Application number : 92303291.6

(22) Date of filing : 13.04.92

(51) Int. Cl.$^5$ : C07C 2/66, C07C 15/14

(30) Priority : 12.04.91 JP 108409/91

(43) Date of publication of application : 14.10.92 Bulletin 92/42

(84) Designated Contracting States : DE FR GB

(71) Applicant : NIPPON OIL CO. LTD.
3-12, Nishi Shinbashi 1-chome
Minato-ku Tokyo (JP)

(72) Inventor : Matsuno, Mitsuo
12-14, Hino 1-chome, Konan-ku
Yokohama-shi, Kanagawa-ken (JP)
Inventor : Enomoto, Toshiyuki
Nisseki Kosugi Apato 933, 2-228 Kosugi-cho
Nakahara-ku, Kawasaki-shi, Kanagawa-ken (JP)

(74) Representative : Myerscough, Philip Boyd
J.A. Kemp & Co. 14 South Square, Gray's Inn
London WC1R 5LX (GB)

(54) **Method of preparing alkylbiphenyls.**

(57) Alkylation of biphenyls or 4-alkylbiphenyls with a catalyst containing phosphorus and having a zeolite structure gives rise to 4-alkyl- and/or 4,4′-dialkylbiphenyls at a high selectivity and in a high yield.

EP 0 508 835 A2

## FIELD OF THE INVENTION

The present invention relates to a method of preparing 4-alkyl- and/or 4,4′-dialkyl biphenyls which are useful as a thermal medium, a solvent for pressure-sensitive copying paper, and a precursor of a liquid crystal molecule.

## DESCRIPTION OF THE PRIOR ART

Alkyl biphenyls are useful not only as a thermal medium, a solvent for pressure-sensitive copying paper or the like but also as a starting material for preparing biphenylcarboxylic acids, hydroxybiphenyl, etc. In particular, various molecules such as 4,4′-biphenyldicarboxylic acid, 4,4′-dihydroxybiphenyl usable as a starting material for liquid crystal polymers or liquid crystal can be derived from 4,4′-alkyl-substituted biphenyls, and hence the latter are important as an intermediate material.

As the method of preparing alkylbiphenyls by alkylation of biphenyl, a method has been known in which biphenyl is alkylated in the presence of a Friedel-Crafts catalyst such as aluminum chloride. This method involves various problems, for example, that it is necessary to perform post-treatment of the catalyst, corrosion of the apparatus occurs, and it is difficult to carry out the process continuously.

Use of a solid acid as the catalyst is advantageous with respect to the aforementioned problems. There have been known various methods of alkylating biphenyl with a solid acid catalyst. For example, Japanese Patent Application Laid-Open No. 156222/1981 discloses a method of preparing alkylbiphenyls rich in meta- and para-forms using a silica-alumina catalyst. Japanese Patent Application Laid-Open No. 122635/1988 describes a method of preparing p,p′-dialkylbiphenyls using a mordenite catalyst. Japanese Patent Application Laid-Open No. 122636/1988 discloses a method in which alkyl-substituted aromatic hydrocarbons are prepared using a mordenite catalyst which has been treated with a fluorine compound. Japanese Patent Application Laid-Open No. 227529/1988 discloses a method for the selective alkylation of biphenyl using a zeolite whose $SiO_2/Al_2O_3$ ratio is 10 or more. Japanese Patent Application Laid-Open No. 190639/1989 describes a method of preparing isopropylbiphenyls using a zeolite catalyst having a pore diameter of about 7 Angstroms. However, all the aforementioned methods are disadvantageous in that they exhibit low selectivity of 4,4′-substituted forms among dialkylbiphenyls prepared.

On the other hand, Japanese Patent Application Laid-Open No. 165531/1981 discloses use of a mordenite catalyst with an increased $SiO_2/Al_2O_3$ ratio in the alkylation of biphenyl to obtain a product containing a 4,4′-substituted form in an increased proportion. However, this method involves some problems from an industrial point of view in that treatment of mordenite with a strong acid in a high concentration is required in order to dealuminate it, which necessitates disposal of waste liquor, causes corrosion of the apparatus used, and so on.

## SUMMARY OF THE INVENTION

It is an object of the present invention to solve the problems involved by the aforementioned conventional methods and provide a method of preparing 4-alkyl and/or 4,4′dialkylbiphenyls at high selectivities.

With view to solving the problems involved by the conventional methods the present inventors have made intensive investigation and as a result it has now been found that use of a specified catalyst makes it possible to obtain 4-alkyl- and/or 4,4′-dialkylbiphenyls or derivatives thereof by alkylating biphenyl or its derivative, and also obtain 4,4′-dialkylbiphenyls or derivatives thereof by alkylating 4-alkylbiphenyls or derivatives thereof. The present invention has been completed based on the discovery.

Accordingly, in one aspect, the present invention provides a methyl of preparing 4-alkyl and/or 4,4′-dialkylbiphenyls, comprising the step of:

alkylating biphenyl or its derivative with an alkylating agent in the presence of a catalyst containing phosphorus and having a zeolite structure.

In another aspect, the present invention provides a method of preparing 4,4′-dialkylbiphenyls, comprising the step of:

alkylating a 4-alkylbiphenyl or its derivative with an alkylating agent in the presence of a catalyst containing phosphorus and having a zeolite structure.

## BRIEF DESCRIPTION OF THE DRAWINGS

Fig. 1 is a graph illustrating results of powder X-ray diffraction analysis of a phosphorus-modified catalyst prepared according to Example 1 of the present invention; and

Fig. 2 is a graph illustrating results of temperature-programmed desorption tests of catalysts prepared in Example 1 and Comparative Example 1 using pyridine.

DETAILED DESCRIPTION OF THE INVENTION

In the method of the present invention, biphenyls can be used as a starting material or compound. Preferred biphenyls are those compounds represented by formula (I) below.

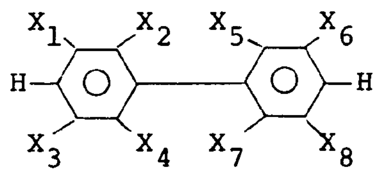

(1)

wherein $X_1$ to $X_8$, which may be same or different, independently represent a hydrogen atom, an alkyl group having up to 8 carbon atoms, a halogen atom or a hydroxyl group.

Specific examples of the preferred biphenyls include isopropylbiphenyl, ethylbiphenyl, butylbiphenyl, etc. Biphenyl is particularly preferred.

As the 4-alkylbiphenyls which can be used as a starting compound in the method of the present invention, there can be cited, for example, 4-alkylbiphenyls which are by-produced upon the alkylation of biphenyl or its derivative for the preparation of 4,4′-dialkylbiphenyls or derivatives thereof.

The 4-alkylbiphenyls which can be used are those compounds represented by formula (II) below.

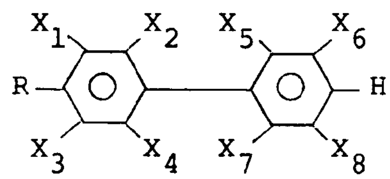

(2)

wherein R represents an alkyl group; and $X_1$ to $X_8$, which may be same or different, independently represent a hydrogen atom, an alkyl group having up to 8 carbon atoms, a halogen atom or a hydroxyl group.

The 4,4′-dialkylbiphenyls which can be used are those compounds represented by formula (III) below.

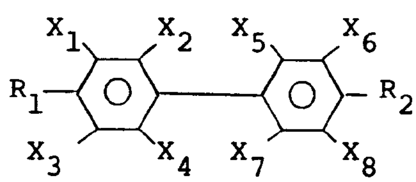

(3)

wherein $R_1$ and $R_2$, which may be same or different, independently represent an alkyl group; and $X_1$ to $X_8$, which may be same or different, independently represent a hydrogen atom, an alkyl group having up to 8 carbon atoms, a halogen atom or a hydroxyl group.

In this invention, an alkyl group is suitably $C_1$-$C_8$, preferably $C_1$-$C_6$.

In the present invention, the alkylating agent used for alkylating the biphenyls or 4-alkylbiphenyls are preferably olefins, aliphatic alcohols, alkyl halides, and polyalkylbenzenes. Olefins and polyalkylbenzenes are particularly preferred. Among olefins, propylene and butene are preferred. As the polyalkylbenzenes, preferred are those which contain 3 or 4 carbon atoms in the pendant alkyl group.

The catalyst used in the present invention for alkylating the biphenyls or 4-alkylbiphenyls is a catalyst which contains phosphorus and has a zeolite structure.

As the phosphorus source for the catalyst used in the present invention, any phosphorus compounds may be used. For example, phosphoric acids and salts thereof, phosphates, phosphoric halides, phosphines and the like can be used. Among them, phosphoric acids and salts thereof are preferred because they can readily be carried using water as a solvent. As the phosphoric acid salt, there can be cited, for example, orthophosphoric acid, metaphosphoric acid, pyrophosphoric acid, and polyphosphoric acid, which is a condensation product of phosphoric acid. These phosphoric acids may be used as mixtures. As the salt of phosphoric acid, there

can be cited, for example, ammonium salts, alkali salts, etc. Ammonium salts are preferred.

Zeolite used for preparing the catalyst employed in the method of the present invention is preferably natural and synthetic zeolites having a pore diameter of 5 Angstroms or more. As such a zeolite, there can be used, for example, faujasite, mordenite, erionite, offretite, L-type zeolite, Ω-type zeolite, super-stabilized zeolite, pentasil-type zeolite, and the like. Among them, faujasite, mordenite, pentasil-type zeolite are preferred, with mordenite being particularly preferred.

The molar ratio of $SiO_2$ to $Al_2O_3$ in zeolite may be of any value. Usually this ratio is 2 to 6 for faujasite, and 30 or more for pentasil-type zeolite. For mordenite, the ratio is usually 10 to 15 but mordenite having a molecular ratio of 15 to 25 or of 25 or more may also be used.

Zeolites have cation exchange ability and there are various zeolites having various cations as counter ions. In the present invention, any cations may be used as the counter cation of the zeolites. Usually, those zeolites containing $Na^+$ ions or $H^+$ ions as the counter ion are readily available. Zeolites containing protons as the counter cation, i.e., acid type zeolites are preferred.

It is preferred that zeolites are preferably subjected to calcination in air or nitrogen as a pre-treatment before they can be modified with phosphorus. Preferred calcination temperature may vary from zeolite to zeolite but generally it is in the range of 250 to 950°C, preferably 500 to 700°C, for mordenite and pentasil-type zeolite, and 200 to 500°C, preferably 250 to 450°C for faujasite. If the calcination temperature is too low, the resulting catalyst is susceptible to adsorbed water while too high a calcination temperature breaks the zeolite structure of the catalyst.

In the present invention, the following methods can be used for preparing a catalyst containing phosphorus and having a zeolite structure.

(1) A method in which zeolite is modified with phosphorus compounds.

(2) A method in which the catalyst is synthesized in copresence of phosphorus compounds in the step of constructing a zeolite structure.

Any method can be used as the method (1) above. For example, an impregnation method and a vapor deposition method may be used.

More specifically, the impregnation method includes:

(i) an adsorption method in which zeolite is dipped in a solution of a phosphorus compound;

(ii) an evaporation method in which zeolite is dipped in a solution of a phosphorus compound, and the solvent is removed by evaporation to dryness;

(iii) a pore-filling method in which zeolite is allowed to adsorb a solution of a phosphorus compound in the same volume as the pore cavity of the zeolite; and

(iv) a spray method in which a solution of a phosphorus compound is sprayed onto zeolite which has been kept in a dry state.

The concentration of the solution of phosphorus compound in which zeolite is dipped is not limited particularly and preferably is 0.01 to 20 M, and more preferably 0.1 to 15 M. However, care must be taken when selecting the concentration in some cases. For example, when dipping zeolite having a low acid resistance in an aqueous solution of orthophosphoric acid, use of a solution having a high concentration will result in a catalyst with a broken zeolite structure. When mordenite or pentasil-type zeolite, which has a high acid resistance is used, the concentration of orthophosphoric acid is preferably 0.05 to 16 M, and more preferably 0.1 to 15 M. Too low a concentration results in inefficient modification.

In the vapor deposition method, modification of zeolite with phosphorus is carried out by contacting zeolite with vapor of phosphorus compounds having high vapor pressures, for example, phosphorus halides, phosphoryl halides, phosphines and the like.

After the modification with phosphorus compounds, the modified zeolite is dries and calcined. The calcination may be carried out under conditions the same as or different from those under which the calcination as pre-treatment is performed. By calcination in air or in an inert gas such as nitrogen, the phosphorus compound is fixed or insolubilized to the zeolite catalyst. This is evident from the fact that even after washing with water, phosphorus atoms still remain in the phosphorus-modified zeolite which has been calcined in air. As compared with the catalyst prior to the modification with phosphorus, the phosphorus-modified catalyst has a decreased acidity, which indicates that change in acidity has occurred during the modification. However, the present invention is limited in no way whether or not the change in acidity has occurred.

The method (2) above can be carried out by hydrothermal synthesis as in the case of the synthesis of ordinary zeolites. The catalyst is a silicoalumino phosphate (so-called SAPO) which has a structure in which zeolite skeleton is partially substituted with phosphorus and for form's sake be represented by formula:

$$(Si_xAl_yP_z)O_2 \ (x + y + z = 1)$$

For details of synthesis and classification, reference is made to J. A. Chem. Soc., 106, 6092 (1984), and Breck, D. W., "Zeolite Molecular Sieves", Wiley, New York (1974), Chapter 4, and Japanese Patent Application

Laid-Open No. 35018/1984. In the practice of the present invention, equivalent results can be obtained by using either the method (1) or method (2) above, and either one of them may be selected freely. However, generally the starting materials are more readily available and operation is easier in the method (1) than in the method (2).

In the present invention, the content of phosphorus is not limited particularly but is preferably 0.02 % by weight, and more preferably 0.1 to 5 % by weight. If the content of phosphorus is more than 8 % by weight, the catalyst has a decreased surface area and decreased conversion, and in addition a decreased selectivity for 4,4′-dialkylbiphenyls in the case of 4-alkylbiphenyls or derivatives. On the other hand, if the content of phosphorus is less than 0.02 % by weight, there is exhibited no effect of increasing the selectivity for 4-alkylbiphenyls or 4,4′-dialkylbiphenyls.

In the present invention, the alkylation of biphenyl or 4-alkylbiphenyls or derivatives thereof in the presence of the catalyst containing phosphorus and having a zeolite structure may be carried out either in a gas phase or liquid phase.

In the case of the gas phase reaction, the reaction temperature is preferably 140 to 400°C, and more preferably 180 t 350°C. The reaction pressure is not limited particularly but is preferably in the range of atmospheric pressure to 200 kg/cm$^2$, and more preferably 2 to 150 kg/cm$^2$.

If the reaction temperature is below 140°C, the reaction rate is low and the conversion decreases. If the reaction temperature exceeds 400°C, isomerization increases considerably to decrease selectivity for 4-alkyl and or 4,4′-dialkylbiphenyls.

Molar proportion of biphenyls or 4-alkylbiphenyls to the alkylating agent is preferably 5/1 to 1/20, and more preferably 1/1 to 1/10.

In the case of the liquid phase reaction, the reaction temperature is preferably 130 to 350°C, and more preferably 170 to 300°C. The reaction pressure is not limited particularly but is preferably in the range of atmospheric pressure to 200 kg/cm$^2$, and more preferably 2 to 150 kg/cm$^2$.

If the reaction temperature is below 130°C, the reaction rate is low and the conversion decreases. If the reaction temperature exceeds 350°C, isomerization increases considerably to decrease selectivity for 4-alkyl and/or 4,4′-dialkylbiphenyls.

Molar proportion of biphenyls or 4-alkylbiphenyls to the alkylating agent is preferably 5/1 to 1/20, and more preferably 1/1 to 1/10. Diluents may be added to the reaction system.

The diluent, there can be cited, for example, decahydronaphthalene, n-decane, undecane, dodecane, tridecane and the like.

The reaction may be carried out either in a continuous system or in a batch system. In the both systems, removal of the catalyst from the product is easy.

The reaction product obtained in the method of the present invention can be removed and purified by distillation or the like.

## EXAMPLES

Hereafter, the method of the present invention will be described more concretely by examples. However, the present invention should not be construed as being limited thereto.

### Example 1 (Preparation of Catalyst)

After calcination in air at 700°C for 2 hours, High Silica Zeolite HSZ650HOA (trade name for acid-type mordenite produced by TOSOH Co., Ltd.; $SiO_2/Al_2O_3$=23) was heated at the reflux temperature of 2M $H_3PO_4$ for 2 hours (adsorption method). Then, after filtration, washing with water and drying at 120°C, the zeolite was calcined at 700°C for 2 hours in air to prepare a catalyst. Measurement of the catalyst by X-ray microanalysis (EPMA) was performed and results obtained are shown in Table 1. From Table 1, it is clear that the catalyst contained phosphorus while $SiO_2/Al_2O_3$ remained unchanged. The content of phosphorus in the catalyst did not change after agitation in water at 90°C for 2 hours. Fig. 1 illustrates results of powder X-ray diffraction analysis. The values of angle of diffraction (2θ) almost correspond to the values obtained for mordenite. From this, it follows that the phosphorus-modified catalyst had mainly a mordenite crystal structure. Fig. 2 illustrates results of temperature-programmed desorption (TPD) using pyridine. From Fig. 2, it can be seen that the modification with phosphorus resulted in decrease in acidity and amount of acid, which indicates that the resulting catalyst became different from mordenite as the starting material.

Example 2 (Preparation of Catalyst)

A catalyst was prepared by repeating the procedures in Example 1 except that 2M $H_3PO_4$ was replaced by 0.33M $H_3PO_4$. Results obtained are shown in Table 1.

Example 3 (Preparation of Catalyst)

A catalyst was prepared by repeating the procedures in Example 1 except that High Silica Zeolite HSZ620HOA (trade name for acid-type mordenite produced by TOSOH Co., Ltd.; $SiO_2/Al_2O_3$ = 15) was used instead. Results obtained are shown in Table 1.

Example 4 (Preparation of Catalyst)

A catalyst was prepared by repeating the procedures in Example 1 except that Zeolon 900H (trade name for acid-type mordenite produced by Morton Co.; $SiO_2/Al_2O_3$ = 12) was used instead. Results obtained are shown in Table 1.

Example 5 (Preparation of Catalyst)

85 % $H_3PO_4$ (2.2 g) was diluted with 20 ml of $H_2O$. HSZ650HOA (15 g) which had been calcined at 700°C for 2 hours in air was impregnated with the solution (Pore-filling method). After drying, the resulting composition was calcined at 700°C for 2 hours in air to prepare a catalyst. Results obtained are shown in Table 1.

Example 6 to 10

In a 900 ml-autoclave were charged 77.1 g (0.5 mol) of biphenyl, 200 ml of decahydronaphthalene and 10 g of the catalyst prepared in Example 1, 2, 3, 4 or 5.

After introducing 42 g (1 mol) propylene in the autoclave, the system was allowed to react at 250°C for 3.5 hours with agitation. The product was analyzed by gas chromatography. Results obtained are shown in Table 2.

Example 11

SAPO-37 was prepared in accordance with the method disclosed in Japanese Patent Application Laid-Open No. 35018/1984. In a mixed solution of 9.2 g of 85 % $H_3PO_4$ and 5.8 g of $H_2O$ was added 5.5 g of boehmite, and the mixture was agitated. To this was added 1.0 g of silica dispersed in 41 g of an aqueous 40 % tetra-n-propylammonium hydride solution, followed by agitation. The resulting mixture was heated in an autoclave at 200°C for 24 hours. The product was centrifuged, washed with water, dried, and then calcined at 500°C for 2 hours.

The catalyst obtained (3 g), 23.1 g (0.15 mol) of biphenyl, 60 ml of decahydronaphthalene were charged in a 300 ml-autoclave, and then 12.6 g (0.3 mol) of propylene was introduced. The mixture was allowed to react at 250°C for 3.5 hours with agitation. The product was analyzed by gas chromatography. Results obtained are shown in Fig. 2.

Example 12

In a 300 ml-autoclave were charged 29.3 g (0.15 mol) of p-isopropylbiphenyl, 60 ml of decahydronaphthalene and 3 g of the catalyst prepared in Example 1. After introducing 3 g of propylene in the autoclave, the mixture was allowed to react at 250°C for 3.5 hours with agitation. The product was analyzed by gas chromatography. Results obtained are shown in Table 3.

Comparative Example 1

The same reaction as in Example 6 was repeated except that the catalyst was replaced by HSZ650HOA calcined at 700°C for 2 hours in air. Results obtained are shown in Table 2. Fig. 2 illustrates results of TPD tests using pyridine.

Comparative Example 2

The same reaction as in Example 6 was repeated except that the catalyst was replaced by HSZ620HOA calcined at 700°C for 2 hours in air. Results obtained are shown in Table 2.

Comparative Example 3

The same reaction as in Example 6 was repeated except that the catalyst was replaced by Zeolon 900H calcined at 700°C for 2 hours in air. Results obtained are shown in Table 2.

Comparative Example 4

The same reaction as in Example 12 was repeated except that the catalyst prepared in Example 1 was replaced by HSZ650HOA calcined at 700°C for 2 hours in air. The composition of the product is shown in Table 3.

Table 1

| Run | Starting Material | Phosphorus Modification | Concentration of Solution (M) | SiO2/Al2O3 | Content of Phosphorus (wt.%) |
|---|---|---|---|---|---|
| Example 1 | HSZ650HOA | Adsorption | 2 | 23 | 1.8 |
| Example 2 | HSZ650HOA | Adsorption | 0.33 | 23 | 1.2 |
| Example 3 | HSZ620HOA | Adsorption | 2 | 15 | 1.1 |
| Example 4 | Zeolon900H | Adsorption | 2 | 12 | 1.0 |
| Example 5 | HSZ650HOA | Pore-filling | 1.1 | 23 | 4.4 |
| Comparative Example 1 | HSZ650HOA | - | - | 23 | 0 |
| Comparative Example 2 | HSZ620HOA | - | - | 15 | 0 |
| Comparative Example 3 | Zeolon900H | - | - | 12 | 0 |

Table 2

| Run | Conversion (%) | Selectivity (%) | | | Composition of mono-substituted product (%) | | | Proportion of 4,4'-disubst. in di-subst. (%) |
|---|---|---|---|---|---|---|---|---|
| | | Mono-Subst. | Di-Subst. | Tri-Subst. | o-form | m-form | p-form | |
| Example 6 | 63 | 41 | 59 | trace | 11 | 27 | 62 | 84 |
| Example 7 | 63 | 40 | 60 | trace | 8 | 27 | 65 | 81 |
| Example 8 | 57 | 45 | 55 | trace | 8 | 24 | 68 | 85 |
| Example 9 | 42 | 54 | 46 | trace | 6 | 19 | 75 | 85 |
| Example 10 | 27 | 70 | 30 | trace | 12 | 16 | 72 | 79 |
| Example 11 | 59 | 45 | 55 | trace | 16 | 24 | 60 | 79 |
| Comparative Example 1 | 72 | 50 | 50 | trace | 35 | 21 | 44 | 44 |
| Comparative Example 2 | 52 | 67 | 33 | trace | 31 | 19 | 50 | 42 |
| Comparative Example 3 | 43 | 73 | 27 | trace | 31 | 23 | 46 | 30 |

Table 3

| Run | Composition (%) | | | | Composition of mono-substituted product(%) | | | Proportion of 4,4'-disubst. in di-subst.(%) |
|---|---|---|---|---|---|---|---|---|
| | Biphenyl | Mono-Subst. | Di-Subst. | Tri-Subst. | o-form | m-form | p-form | |
| Example 12 | 5 | 40 | 55 | trace | 1 | 6 | 93 | 88 |
| Comparative Example 4 | 8 | 32 | 60 | trace | 15 | 41 | 44 | 45 |

## Claims

1. A method of preparing a 4-alkyl- and/or 4,4′-dialkylbiphenyl, which method comprises:
alkylating a biphenyl with an alkylating agent in the presence of a catalyst containing phosphorus and having a zeolite structure.

2. A method of preparing a 4,4′-dialkylbiphenyl, which method comprises:
alkylating a 4-alkylbiphenyl with an alkylating agent in the presence of a catalyst containing phosphorus and having a zeolite structure.

3. A method according to claim 1 or 2, wherein said catalyst contains phosphorus in an amount of from 0.02 to 8 % by weight.

4. A method according to claim 1, 2 or 3, wherein said catalyst is prepared from natural or synthetic zeolite having a pore diameter of 5 Å or more.

5. A method according to claim 4, wherein said zeolite is faujasite, mordenite, erionite, offretite, L-type zeolite, Ω-type zeolite, superstabilized zeolite or pentasil-type zeoite.

6. A method according to claim 4 or 5, wherein said zeolite contains Na+ ion or $H^+$ ion as counter cation.

7. A method according to any one of the preceding claims, wherein the alkylation is carried out in the vapour phase at a temperature of from 140 to 400°C.

8. A method according to any one of the preceding claims, wherein the alkylation is carried out in the liquid phase at a temperature of from 130 to 350°C.

9. A method according to any one of the preceding claims, wherein said biphenyl or 4-alkylbiphenyl and said alkylating agent are used in a molar ratio of from 5/1 to 1/20.

10. A method according to any one of the preceding claims, wherein the alkylation is carried out in the presence of a diluent.

11. A method according to claim 10, wherein said diluent is decahydronaphthalene, n-decane, n-undecane, n-dodecane or n-tridecane.

12. A method according to any one of the preceding claims, wherein said alkylating agent is an olefin, an aliphatic alcohol, an alkyl halide, or a polyalkylbenzene.

13. A method according to claim 12, wherein said alkylating agent is propylene or butene.

14. A method according to claim 12, wherein said alkylating agent is a polyalkylbenzene having a $C_3$-$C_4$ alkyl group.

FIG. 1

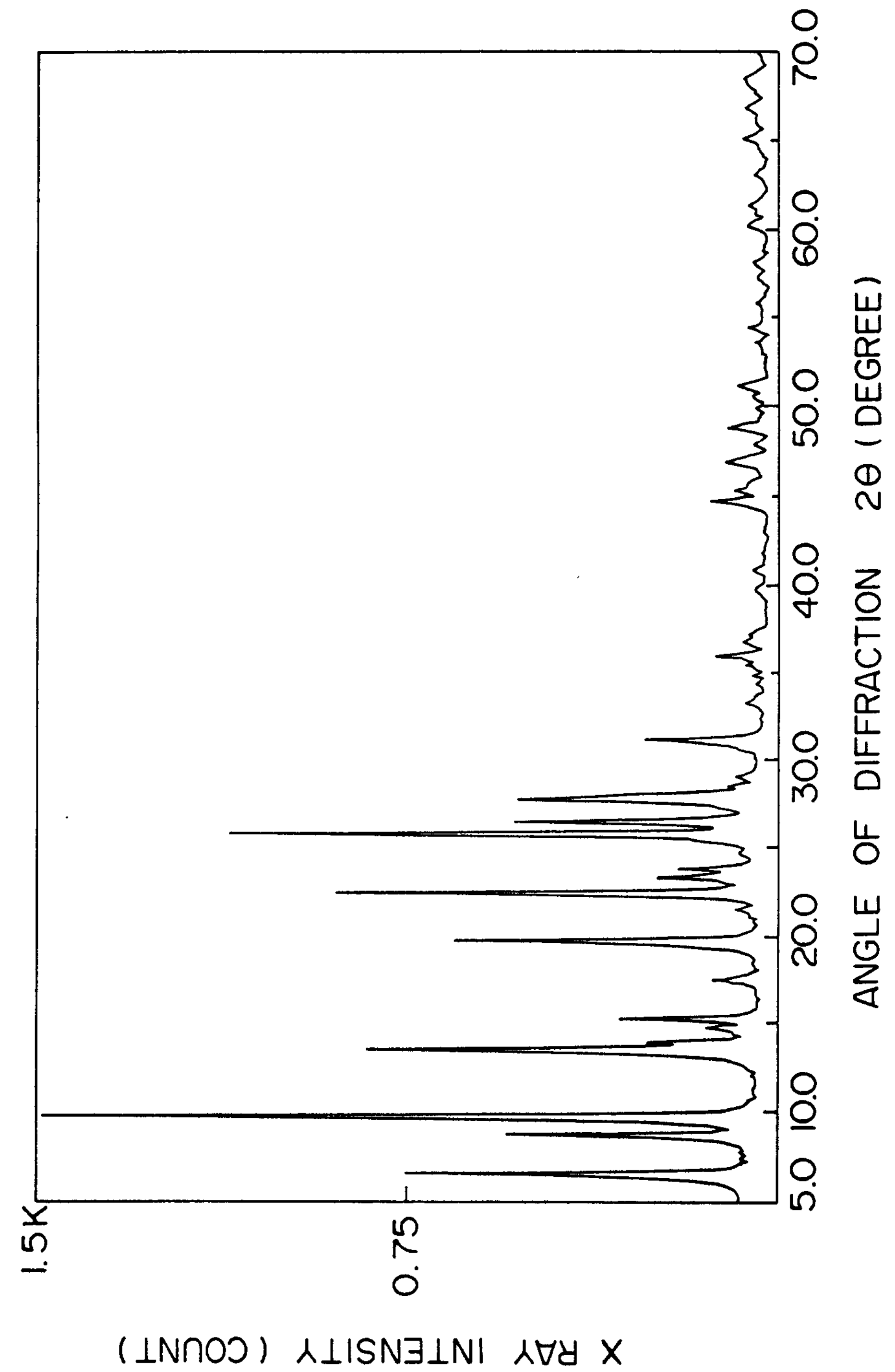
1.5K
0.75
X RAY INTENSITY (COUNT)
5.0
10.0
20.0
30.0
40.0
50.0
60.0
70.0
ANGLE OF DIFFRACTION 2θ (DEGREE)

FIG. 2

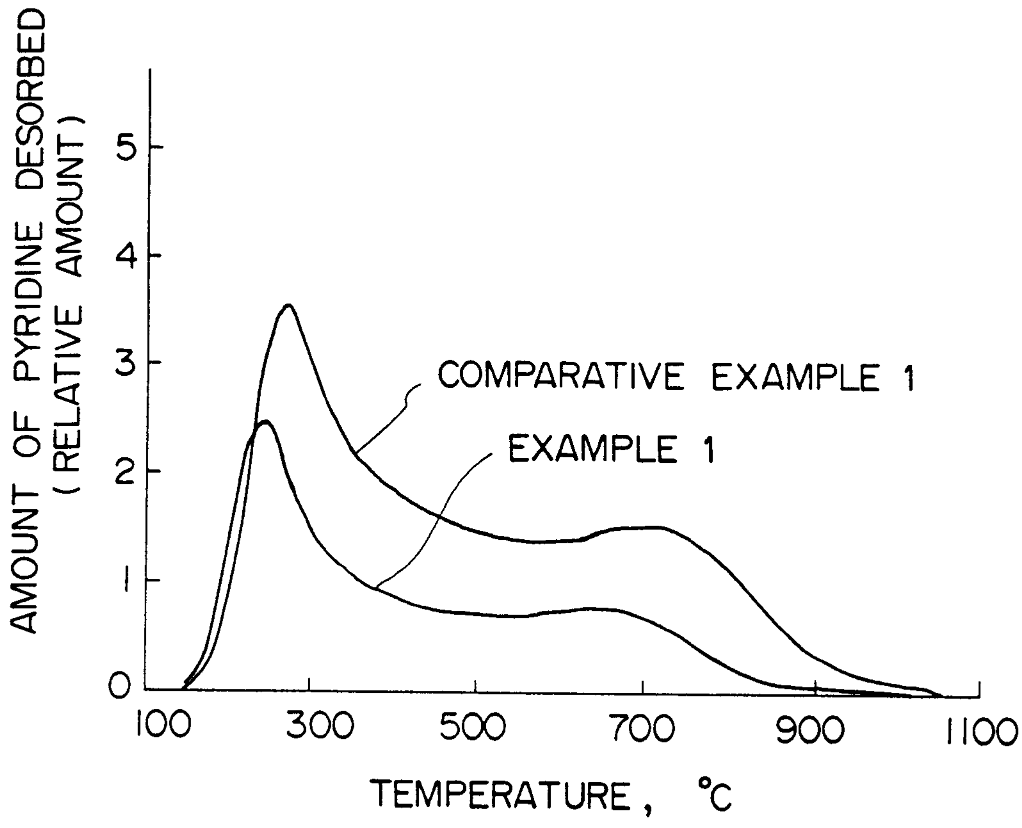
AMOUNT OF PYRIDINE DESORBED
(RELATIVE AMOUNT)
0
1
2
3
4
5
100
300
500
700
900
1100
TEMPERATURE, °C
COMPARATIVE EXAMPLE 1
EXAMPLE 1